# FASCICULE DE BREVET EUROPEEN

(11) **EP 0 643 136 B1**
(45) Date de publication et mention de la délivrance du brevet: **31.10.2001**
(21) Numéro de dépôt: 94112913.2
(22) Date de dépôt: 19.08.1994
(51) Int. Cl.: C12N 15/62, C07K 14/315, C12P 21/02, A61K 7/16, A61K 7/00, A23C 9/123, A23B 4/22, A61K 38/16, A23L 3/3571

(54) **Bactériocine de Streptococcus thermophilus**
Bacteriocine aus Streptococcus thermophilus
Bacterion of Streptococcus thermophilus

(30) Priorité: 03.09.1993 CH 262893
(43) Date de publication de la demande: 15.03.1995
(73) Titulaire: SOCIETE DES PRODUITS NESTLE S.A., 1800 Vevey (CH)
(72) Inventeur: Germond, Jacques Edouard, CH-1023 Crissier (CH); Marciset, Olivier, CH-1005 Lausanne (CH); Mollet, Beat, CH-1074 Mollie-Margot (CH)
(74) Mandataire: Wavre, Claude-Alain

(56) Documents cités:
- EP-A- 0 443 543
- DEUTSCHE MOLKEREI-ZEITUNG, vol.105, no.15, 1984 pages 460 - 464 SMACZNY, T. ET AL. 'Säuerungstörungen in der Joghurt-, Bioghurt -und Biogarde -Produktion, bedingt durch Bacteriocine und Bakteriophagen von Streptococcus thermophilus'
- ABSTRACTS OF THE ANNUAL MEETING OF THE AMERICAN SOCIETY FOR MICROBIOLOGY, Mai 1993, WASHINGTON US page 344 WARD, D. J. ET AL. 'Bacteriocin production in Streptococcus thermophilus'

### LISTE DE SEQUENCES

(1) INFORMATION GENERALE:
   (i) DEPOSANT:
      (A) NOM: SOCIETE DES PRODUITS NESTLE S.A.
      (B) RUE: Case postale 353
      (C) VILLE: Vevey
      (E) PAYS: Suisse
      (F) CODE POSTAL: 1800
      (G) TELEPHONE: (021) 924 47 60
      (H) TELECOPIE: (021) 924 28 80
   (ii) TITRE DE L' INVENTION: Bacteriocines de Streptococcus thermophilus
   (iii) NOMBRE DE SEQUENCES: 8
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
   (vi) DONNEES DE LA DEMANDE ANTERIEURE:
      (A) NUMERO DE DEPOT: CH 2628/93-7
      (B) DATE DE DEPOT: 03-SEP-1993
(2) INFORMATION POUR LA SEQ ID NO: 1:
   (i) CARACTERISTIQUES DE LA SEQUENCE:
      (A) LONGUEUR: 62 acides aminés
      (B) TYPE: acide aminé
      (D) CONFIGURATION: linéaire
   (ii) TYPE DE MOLECULE: protéine
   (vi) ORIGINE:
      (A) ORGANISME: Streptococcus thermophilus
      (B) SOUCHE: CNCM I-1351
   (xi) DESCRIPTION DE LA SEQUENCE: SEQ ID NO: 1:
   Tyr Ser Gly Lys Asp Cys Leu Lys Asp Met Gly Gly Tyr Ala Leu Ala
   1               5                   10                  15
   Gly Ala Gly Ser Gly Ala Leu Trp Gly Ala Pro Ala Gly Gly Val Gly
               20                  25                  30
   Ala Leu Pro Gly Ala Phe Val Gly Ala His Val Gly Ala Ile Ala Gly
           35                  40                  45
   Gly Phe Ala Cys Met Gly Gly Met Ile Gly Asn Lys Phe Asn
       50                  55                  60
(2) INFORMATION POUR LA SEQ ID NO: 2:
   (i) CARACTERISTIQUES DE LA SEQUENCE:
      (A) LONGUEUR: 43 acides aminés
      (B) TYPE: acide aminé
      (D) CONFIGURATION: linéaire
   (ii) TYPE DE MOLECULE: protéine
   (vi) ORIGINE:
      (A) ORGANISME: Streptococcus thermophilus
      (B) SOUCHE: CNCM I-1351
   (xi) DESCRIPTION DE LA SEQUENCE: SEQ ID NO: 2:
   Gln Ile Asn Trp Gly Ser Val Val Gly His Cys Ile Gly Gly Ala Ile
   1               5                   10                  15
   Ile Gly Gly Ala Phe Ser Gly Gly Ala Ala Ala Gly Val Gly Cys Leu
               20                  25                  30
   Val Gly Ser Gly Lys Ala Ile Ile Asn Gly Leu
           35                  40
(2) INFORMATION POUR LA SEQ ID NO: 3:
   (i) CARACTERISTIQUES DE LA SEQUENCE:
      (A) LONGUEUR: 770 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Streptococcus thermophilus
      (B) SOUCHE: CNCM I-1351
   (ix) CARACTERISTIQUE ADDITIONELLE:
      (A) NOM/CLE: CDS
      (B) EMPLACEMENT: 221..475
   (ix) CARACTERISTIQUE ADDITIONELLE:
      (A) NOM/CLE: sig_peptide
      (B) EMPLACEMENT: 221..289
   (ix) CARACTERISTIQUE ADDITIONELLE:
      (A) NOM/CLE: mat_peptide
      (B) EMPLACEMENT: 290..475
      (D) AUTRES RENSEIGNEMENTS: /function= "code pour la thermophiline 1"
   (ix) CARACTERISTIQUE ADDITIONELLE:
      (A) NOM/CLE: CDS
      (B) EMPLACEMENT: 495..686
   (ix) CARACTERISTIQUE ADDITIONELLE:
      (A) NOM/CLE: sig _peptide
      (B) EMPLACEMENT: 495..557
   (ix) CARACTERISTIQUE ADDITIONELLE:
      (A) NOM/CLE: mat_peptide
      (B) EMPLACEMENT: 558..686
      (D) AUTRES RENSEIGNEMENTS: /function= "code pour la thermophiline 2"
   (xi) DESCRIPTION DE LA SEQUENCE: SEQ ID NO: 3:
   AATGGCACGA ACGTCCTGAA TGGTTAAAAG ATATTTCGGA TCTTCCTAAA AAATACATAC      60
   TGAACGGTCG CTTTCCCTTC TTGAATGGTA AAATTTTCCC ATTAGGAAAG TTAAATGACT     120
   GTTCAAGAAA TGGGGAAATT ATTTTTTGAA GTAGTGCTAT ACTAGACTTG TCAAGGTTGC     180
   AACCCGACAA AATAAAAATA TTAGGTAGGA GATATTTACA ATG AAT ACA ATA ACT      235
                                               Met Asn Thr Ile Thr
                                               -23         -20
   ATT TGT AAA TTT GAT GTT TTA GAT GCT GAA CTT CTT TCG ACA GTT GAG      283
   Ile Cys Lys Phe Asp Val Leu Asp Ala Glu Leu Leu Ser Thr Val Glu
               -15                 -10                 -5
   GGT GGA TAC TCT GGT AAG GAT TGT TTA AAA GAC ATG GGA GGA TAT GCA      331
   Gly Gly Tyr Ser Gly Lys Asp Cys Leu Lys Asp Met Gly Gly Tyr Ala
            1               5                  10
   TTG GCA GGA GCT GGA AGT GGA GCT CTG TGG GGA GCT CCA GCA GGA GGT      379
   Leu Ala Gly Ala Gly Ser Gly Ala Leu Trp Gly Ala Pro Ala Gly Gly
    15                  20                  25                  30
   GTT GGA GCA CTT CCA GGT GCA TTT GTC GGA GCT CAT GTT GGG GCA ATT      427
   Val Gly Ala Leu Pro Gly Ala Phe Val Gly Ala His Val Gly Ala Ile
                    35                  40                  45
   GCA GGA GGC TTT GCA TGT ATG GGT GGA ATG ATT GGT AAT AAG TTT AAC      475
   Ala Gly Gly Phe Ala Cys Met Gly Gly Met Ile Gly Asn Lys Phe Asn
                50                  55                  60
   TAAGGAAGGA GTTTATATC ATG AAG CAG TAT AAT GGT TTT GAG GTT CTA CAT      527
                        Met Lys Gln Tyr Asn Gly Phe Glu Val Leu His
                        -21 -20                 -15
   GAA CTT GAC TTA GCA AAT GTA ACT GGC GGT CAA ATT AAT TGG GGA TCA      575
   Glu Leu Asp Leu Ala Asn Val Thr Gly Gly Gln Ile Asn Trp Gly Ser
   -10                 -5                   1               5
   GTT GTA GGA CAC TGT ATA GGT GGA GCT ATT ATC GGA GGT GCA TTT TCA        623
   Val Val Gly His Cys Ile Gly Gly Ala Ile Ile Gly Gly Ala Phe Ser
               10                  15                  20
   GGA GGT GCA GCG GCT GGA GTA GGA TGC CTT GTT GGG AGC GGA AAG GCA        671
   Gly Gly Ala Ala Ala Gly Val Gly Cys Leu Val Gly Ser Gly Lys Ala
           25                  30                  35
   ATC ATA AAT GGA TTA TAAAAGTCTT TTATCGCTTT TATTATTCAT AATTCCCCTT        726
   Ile Ile Asn Gly Leu
        40
   GTAGTTATAC TAATCGTTCT TCGAAAGAAT AATCAGAAAC TAAT                       770
(2) INFORMATION POUR LA SEQ ID NO: 4:
   (i) CARACTERISTIQUES DE LA SEQUENCE:
      (A) LONGUEUR: 85 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Met Asn Thr Ile Thr Ile Cys Lys Phe Asp Val Leu Asp Ala Glu Leu
   -23         -20                 -15                 -10
   Leu Ser Thr Val Glu Gly Gly Tyr Ser Gly Lys Asp Cys Leu Lys Asp
           -5                  1               5
   Met Gly Gly Tyr Ala Leu Ala Gly Ala Gly Ser Gly Ala Leu Trp Gly
    10                  15                  20                  25
   Ala Pro Ala Gly Gly Val Gly Ala Leu Pro Gly Ala Phe Val Gly Ala
                   30                  35                  40
   His Val Gly Ala Ile Ala Gly Gly Phe Ala Cys Met Gly Gly Met Ile
               45                  50                  55
   Gly Asn Lys Phe Asn
           60
(2) INFORMATION POUR LA SEQ ID NO: 5:
   (i) CARACTERISTIQUES DE LA SEQUENCE:
      (A) LONGUEUR: 64 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 5:
   Met Lys Gln Tyr Asn Gly Phe Glu Val Leu His Glu Leu Asp Leu Ala
   -21 -20                 -15                 -10
   Asn Val Thr Gly Gly Gln Ile Asn Trp Gly Ser Val Val Gly His Cys
    -5                   1               5                  10
   Ile Gly Gly Ala Ile Ile Gly Gly Ala Phe Ser Gly Gly Ala Ala Ala
               15                  20                  25
   Gly Val Gly Cys Leu Val Gly Ser Gly Lys Ala Ile Ile Asn Gly Leu
           30                  35                  40
(2) INFORMATION POUR LA SEQ ID NO: 6:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: OUI
   (xi) DESCRIPTION DE LA SEQUENCE: SEQ ID NO: 6:
   GAYATGGGNG GNTAYGC                                                 17
(2) INFORMATION POUR LA SEQ ID NO: 7:
   (i) CARACTERISTIQUES DE LA SEQUENCE:
      (A) LONGUEUR: 17 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: OUI
   (xi) DESCRIPTION DE LA SEQUENCE: SEQ ID NO: 7:
   GCTATNGCNC CNACGTG                                                 17
(2) INFORMATION POUR LA SEQ ID NO: 8:
   (i) CARACTERISTIQUES DE LA SEQUENCE:
      (A) LONGUEUR: 128 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Streptococcus thermophilus
      (B) SOUCHE: CNCM I-1351
   (xi) DESCRIPTION DE LA SEQUENCE: SEQ ID NO: 8:
   GATTGTTTAA AAGACATGGG AGGATATGCA TTGGCAGGAG CTGGAAGTGG AGCTCTGTGG     60
   GGAGCTCCAG CAGGAGGTGT TGGAGCACTT CCAGGTGCAT TTGTCGGAGC TCATGTTGGG    120
   GCAATTGC                                                             128

## Description

La présente invention a pour objet deux bactériocines de *Streptococcus (S.) thermophilus*, une souche de *S.thermophilus* productrice de ces bactériocines, un procédé de production de ces bactériocines à partir de cette souche, ainsi que des utilisations de ces bactériocines et/ou de cette souche dans la préparation de produits alimentaires ou de produits cosmétiques.

### ETAT DE LA TECHNIQUE

Une bactériocine est une substance antibactérienne ou agent actif contre les bactéries comprenant une partie protéique impliquée dans l'effet antibactérien ou effet antibiotique. Une bactériocine présente généralement un spectre d'action ou spectre d'inhibition étroit souvent limité aux espèces proches de l'espèce de la bactérie qui la produit.

Actuellement, on connaît quatre bactériocines de *S. thermophilus*.

La première présente en particulier un poids moléculaire de 10 à 20 kD, une thermolabilité à 90°C, et une sensibilité à la pepsine (Smaczny et al., Deutsche Molkerei-Zeitung, 105:15, 460-464, 1984).

La deuxième, décrite principalement par son spectre d'inhibition de bactéries dans EP 443543, présente notamment la capacité d'inhiber la croissance de bactéries du genre staphylocoque et pseudomonas, et l'incapacité d'inhiber la croissance de bactéries du genre lactocoques et entérocoques, et de l'espèce *Bacillus cereus*.

La troisième, décrite par Pulusani et al. (J. of Food Science, 44:2, 575-578, 1979), inhibe fortement Pseudomonas, n'est pas sensible à la pepsine, et contient des résidus de sucres.

Enfin la quatrième, décrite par Cilano et al. (Microbiologie-Aliment-Nutrition, 8, 21-30, 1990), n'est pas sensible à la pepsine, contient des résidus de sucres et ne passe pas à travers une membrane de porosité de 100 kD.

Or, *S.thermophilus* présente une importance majeure dans le domaine alimentaire, étant notamment impliquée dans la préparation de produits laitiers tels que les yogourts et certains fromages, par exemple. De plus il existe très peu de bactériocines actives à la fois contre les *Bacilli*, les *Clostridia*, et les *Listéria*. Il pourrait être donc des plus utiles, autrement dit il existe un besoin de disposer d'une plus large palette de bactériocines produites par des représentantes de cette espèce afin de disposer notamment d'un plus large spectre d'action antibactérien, en particulier dans le contexte de ce type de produits.

La présente invention a pour but de répondre à ce besoin.

### RESUME DE L'INVENTION

Un des objets de la présente invention est la caractérisation de la séquence en acides aminés de deux nouvelles bactériocines de *S.thermophilus*, ainsi que leur peptide signal permettant leur excrétion.

Les séquences nucléotidiques codant pour ces deux bactériocines sont également un autre objet de l'invention.

Un autre objet de l'invention, est la souche de *S.thermophilus* CNCM I-1351 décrite ci-après, capable de produire les deux bactériocines selon l'invention.

Le procédé de production d'un extrait d'au moins une bactériocine selon la présente invention est encore un autre objet de la présente invention.

Enfin, un dernier objet de la présente invention est l'utilisation des bactériocines selon l'invention et l'utilisation de leur séquence nucléique, ainsi que leur séquence signale.

### DESCRIPTION DETAILLEE DE L'INVENTION

On a isolé une souche *S. thermophilus* CNCM I-1351 d'un produit laitier fermenté de Tchécoslovaquie et on a constaté avec surprise qu'elle présente la propriété remarquable d'inhiber la croissance d'une large palette de bactéries. Cette souche a été déposée le 05.08.93, selon le Traité de Budapest, à la Collection Nationale de Cultures de Microorganismes, INSTITUT PASTEUR, 25, Rue du Docteur Roux, F-75724 PARIS CEDEX 15, France où elle a donc reçu le No I-1351.

Des détails sur cette souche concernant notamment sa morphologie, la fermentation des sucres et autres sont donnés ci-après:

### Morphologie:

- Coques formant des chaînettes, non flagellés. Pas de formation de spores.
- Microorganismes Gram positifs, catalase négatifs et anaérobes facultatifs.

### Fermentation des sucres:

Production d'acide lactique à partir de D-glucose, lactose, saccharose, raffinose. Pas de production d'acide lactique à partir de mannose, fructose, galactose.

### Autres:

Souche produisant au moins deux bactériocines, une protéine d'immunité aux bactériocines, et des exopolysaccharides ayant des propriétés texturantes.

Le surnageant de culture de la souche CNCM I-1351 présente donc un spectre d'activité anti-bactéries relativement large. Parmi les bactéries sensibles à ce surnageant, on peut comprendre *Streptococcus thermophilus, Lactococcus lactis, Lactococcus lactis biovar diacetylactis, Lactococcus cremoris, Enterococcus faecalis, Enterococcus faecium, Lactobacillus fermentum, Lactobacillus helveticus, Lactobacillus bulgaricus, Lactobacillus acidophilus, Lactobacillus brevis, Leuconostoc cremoris, Leuconostoc mesenteroides, Bifidobacterium breve, Bifidobacterium longum, Bifidobacterium bifidum, Bifidobacterium infantis, Propionibacterium, Listeria innocua, Listeria momocytogenes, Micrococcus varians,* et les spores et les cellules végétatives de *Clostridium botulinum, Clostridium tyrobutyricum, Clostridium bifermentans, Clostridium sporogenes, Bacillus subtilis, Bacillus pumilus et Bacillus cereus*, par exemple (Bactéries lactiques, vol 1, 1994, édition Lorica).

On a pu ensuite isoler de cette souche CNCM I-1351 deux facteurs protéiques appelés bactériocines, responsables de cette activité antibactérienne.

A cet effet, la première bactériocine selon l'invention nommée dans cet exposé "thermophiline 1", présente la séquence SEQ ID NO:1 décrite dans la liste de séquences ci-après.

De plus, on peut envisager que cette bactériocine puisse présenter une activité antibactérienne ayant un spectre plus large ou plus spécifique pour un genre ou une espèce bactérienne que celui présenté par la thermophiline 1, lorsque celle-ci possède une séquence qui diffère de la séquence SEQ ID NO: 1 par une substitution, une délétion et/ou une insertion d'au moins un acide aminé, par exemple. En effet, il est déjà connu par EP521240 que la nisine Z présente un spectre d'activité plus intéressant que la nisine A, alors que celle-ci ne diffère de la nisine A que par une subsitution d'un acide aminé.

La deuxième bactériocine selon l'invention, nommée dans cet exposé "thermophiline 2", présente la séquence SEQ ID NO:2 décrite dans la liste de séquences ci-après. On peut aussi envisager que cette bactériocine puisse présenter une activité antibactérienne lorsque celle-ci présente une séquence qui diffère de la séquence SEQ ID NO:2 par une substitution, une délétion et/ou une insertion d'un acide aminé, par exemple.

En outre, les séquences nucléotidiques codant pour la thermophiline 1 et la thermophiline 2 sont également un autre objet de la présente invention, car elles peuvent être chacune utilisées pour conférer par transformation à des bactéries, des levures ou des plantes par exemple, une capacité d'inhiber certaines bactéries. Ces séquences nucléotidiques peuvent être ainsi relativement variables du fait de la dégénérescence du code génétique, et peuvent notamment être comprises dans un opéron de la souche CNCM I-1351 ayant la séquence nucléique SEQ ID NO:3 décrite dans la liste de séquences ci-après.

En particulier, on peut utiliser la séquence nucléique comprenant les nucléotides 221 à 475 de la séquence SEQ ID NO:3, qui code pour la thermophiline 1 avec son peptide signal. Cependant, il est plus intéressant d'utiliser uniquement la séquence codant pour le peptide signal, du nucléotide 221 à 288 de la séquence SEQ ID NO:3, pour la fusionner à un gène d'intérêt, de manière à pouvoir permettre l'excrétion par une souche de *Streptococcus thermophilus* de la protéine codée par ce gène d'intérêt De même, il est plus intéressant d'utiliser uniquement la séquence codant pour la thermophiline 1 excrétée, du nucléotide 289 à 475 de la séquence SEQ ID NO:3, pour pouvoir la fusionner à une séquence signale dans un plasmide d'expression, par exemple, de manière à l'exprimer dans un autre microorganisme que *S. thermophilus*.

De même, on peut utiliser la séquence nucléique comprenant les nucléotides 495 à 686 de la séquence SEQ ID NO:3, qui code pour la thermophiline 2 avec son peptide signal. Cependant, il est plus intéressant d'utiliser uniquement la séquence codant pour le peptide signal, du nucléotide 495 à 557 de la séquence SEQ ID NO:3, pour pouvoir permettre l'excretion par une souche de *Streptococcus thermophilus* de toute protéine fusionnée à ce peptide. De même, il est plus intéressant d'utiliser uniquement la séquence codant pour la thermophiline 2 excrétée, du nucléotide 558 à 686 de la séquence SEQ ID NO:3, pour pouvoir la fusionner à une séquence signal dans un plasmide d'expression, par exemple, de manière à l'exprimer dans un autre microorganisme que *S. thermophilus*.

Enfin, comme on a observé que certaines souches de *S. thermophilus*, autres que la souche CNCM I-1351, présentent un spectre d'inhibition similaire à celui présenté par la souche CNCM I-1351 et une résistance à cette dernière (notamment les souches Sfi12 et 25 décrites ci-après), il est fort probable que ces souches puissent produire au moins une des bactériocines de la présente invention, en même temps qu'une protéine d'immunité conférant cette résistance.

Dans le procédé de production d'un extrait comprenant au moins une bactériocine selon la présente invention, on cultive une souche de *S.thermophilus* productrice d'au moins une desdites bactériocines dans un milieu et dans des conditions favorables à la croissance de *S.thermophilus* jusqu'à ce que le milieu contienne de 10⁷-10⁹ germes de la souche par ml, on centrifuge la culture obtenue, puis on prépare un extrait du surnageant comprenant au moins une desdites bactériocines.

Pour produire cet extrait, la souche de *S.thermophilus* productrice d'au moins une desdites bactériocines selon la présente invention, notamment la souche *S.thermophilus* CNCM I-1351, peut donc être cultivée dans un milieu et dans des conditions favorables à la croissance de *S.thermophilus*. On peut la cultiver notamment dans un milieu MSK (lait de vache écrémé supplémenté avec de l'extrait de levure) ou dans un milieu HJ (perméat d'ultrafiltration de lait de vache supplémenté avec de l'extrait de levure et du soytone), par exemple. On la cultive de préférence dans un milieu sélectif pour les *Streptococci,* tel que le milieu M 17 décrit par P.E. Terzaghi et al., J. Appl. Microbiol, **29**, 807-813 (1975), supplémenté de 0,5-2% d'un sucre fermentable par *S.thermophilus*, notamment le saccharose, le lactose ou le glucose, par exemple.

Un tel milieu peut être préparé en mélangeant 95 ml d'un milieu de base et 5 ml d'une solution à 10 g de sucre fermentescible pour 100 ml d'eau, la solution de sucre fermentescible et le milieu de base ayant été stérilisés chacun séparément à 121°C durant 15 min et le milieu de base ayant été préparé en dissolvant les composants suivants dans 950 ml d'eau en ébullition:
- hydrolysat trypsique de caséine 2,5 g
- hydrolysat pepsique de viande 2,5 g
- hydrolysat papaïque de soya 5,0 g
- extrait de levure 2,5 g
- extrait de viande 5,0 g
- beta-glycerophospahate 19 g
- sulfate de Mg 0,25 g
- acide ascorbique 0,5 g

On peut cultiver ladite souche dans ledit milieu favorable à la croissance de *S.thermophilus* à 37-48°C, durant 2-8 h, par exemple, jusqu'à ce que le milieu contienne environ 10⁷-10⁹ germes de la souche par ml, une valeur de environ 10⁸ germes/ml correspondant d'une part à une densité optique du milieu mesurée à 600nm (OD₆₀₀) de environ 3,6 et d'autre part à la concentration atteinte dans un lait de vache au moment où il coagule sous l'effet de l'acidification produite par la souche cultivée.

Pour préparer un extrait brut dudit surnageant, on peut utiliser toute méthode de précipitation adéquate telle qu'une précipitation à l'acide trichloracétique, un "salting out" ou une précipitation au solvant, par exemple. De préférence, pour préparer cet extrait brut, on ajuste le pH du surnageant à 1,0-2,0 avec H₃PO₄, on écarte un précipité, et l'on réalise une ou plusieurs précipitations successives à l'acide trichloracétique suivies chacune d'une remise en suspension aqueuse à l'acide trifluoroacétique.

Une utilisation des bactériocines et/ou d'une souche de *Streptococcus thermophilus* productrice de ces bactériocines selon la présente invention est prévue dans la préparation de produits alimentaires ou de produits cosmétiques.

On peut utiliser en particulier une culture de ladite souche de *Streptococcus thermophilus* comme levain dans la préparation de fromages, notamment de fromages du type mozzarella (pour éviter les trous produits par *Bacilllus polymixa* dont les spores survivent à la fermentation), du type suisse (tel que Gruyère ou Emmental, pour lutter contre la contamination par *Clostridium tyrobutyricum*), du type vacherin (pour lutter contre la contamination par *Listeria monocytogenes*), et du type séré, ou dans la préparation de laits acidifiés, notamment de yogourt ou de lait en poudre pour formules infantiles, par exemple.

En particulier, on peut cultiver dans du lait ladite souche de *Streptococcus thermophilus* en combinaison avec une souche de *Lactobacillus bulgaricus* moyennement sensible à la thermophiline (par exemple la souche YL5 décrite ci-après), pour éviter la postacidification du yogourt due au *L. bulgaricus*.

On peut utiliser également lesdites bactériocines, notamment sous forme d'extrait brut ou purifié, ou ladite souche comme additif ou agent actif contre des bactéries pathogènes, notamment dans la préparation de produits de viande tels que des mousses, comme agent actif contre la croissance de spores de clostridia, notamment *Clostridium botulinum*, ou dans la préparation de crèmes ou lotions, comme agent actif contre des bactéries pathogènes de la peau, ou encore dans la préparation de produits de l'hygiène buccale, comme agent actif contre des bactéries pathogènes de la cavité buccale, notamment contre *Streptococcus sobrinus*, par exemple.

Les bactériocines selon la présente invention sont caractérisées plus en détail ci-après par l'intermédiaire de différentes données microbiologiques, biochimiques et génétiques illustrant leurs propriétés. Les pourcentages sont données en poids.

### Unité d'activité antibactérienne - "Agar Well-Test"

Dans le cadre du présent exposé, on définit l'activité antibactérienne en termes d'unités arbitraires.

Une unité arbitraire (ua) est définie comme l'inverse du taux de la plus grande dilution à laquelle un échantillon montre encore une activité antibactérienne dans le test connu de l'homme du métier sous la désignation d' "agar well test", expression anglaise qui signifie littéralement test du puits creusé dans l'agar.

Un échantillon standard d'un surnageant d'une culture de *S. thermophilus* selon la présente invention préparée dans les conditions standard illustrées à l'Exemple 1, présente typiquement une activité de 32 ua pour un volume de 70 µl. Cela signifie donc une activité de 460 ua/ml.

Un extrait brut standard de la bactériocine, obtenu à partir du surnageant de culture illustré à l'Exemple 1 par une clarification suivie par deux précipitations successives à l'acide trichloracétique suivies chacune d'une remise en suspension aqueuse à l'acide trifluoroacétique, présente typiquement une activité de environ 1,4x10⁵ ua/ml.

C'est à l'aide dudit agar well test que l'on détermine si un échantillon montre encore une activité antibactérienne à un taux de dilution donné.

Pour ce faire, on verse dans une boîte de Petri 35 ml de milieu M 17 auquel on ajoute 1% de saccharose et 1,5% d'agar.

On inocule 5 ml de milieu M17 auquel on ajoute 1% de saccharose et 0,75% d'agar avec 5 µl d'une culture préparée durant la nuit précédente d'une souche de *S.thermophilus* typiquement sensible à la présente bactériocine (indicateur type), en l'occurrence la souche Sfi3, par exemple.

On verse les 5 ml sur les 35 ml et l'on met à sécher durant 15 min sous flux laminaire. On perce des trous de 5 mm de diamètre sur le milieu de culture.

On déverse les échantillons à tester dans les trous, à raison de 70 µl par trou. On incube durant 6 h en anaérobiose à 42°C. Durant cette incubation, la souche indicateur type a poussé et des halos d'inhibition sont visibles. Le taux de dilution auquel un échantillon ne présente plus d'activité antibactérienne est le taux de dilution à partir duquel on ne distingue plus de halo d'inhibition.

### Inactivation par des enzymes

A l'aide dudit agar well test, sur agar inoculé avec ladite souche indicateur type comme décrit ci-dessus, on détermine si les présentes bactériocines sont inactivées ou non par différentes enzymes.

Pour toutes les enzymes utilisées sauf pour la lipase, on ajoute 1 µg/ml à 10mg/ml d'enzyme audit extrait brut standard dilué 33x dans le tampon recommandé par le fournisseur d'enzyme, de manière à obtenir des échantillons de 70 µl à 300 ua. On laisse ensuite agir l'enzyme 30 min, à la température recommandée par le fournisseur, avant de déposer le tout dans le puits de l'agar well test.

Par contre, pour la lipase commerciale, on ajoute d'abords 1 µl d'un mélange d'inhibiteurs (EDTA 1,25M; 0,25% pepstatin A (p4265 Sigma); 0,25% E-64 (E3132 Sigma); 0,25% A protinin (A1153 Sigma)) à 100 µl d'une solution comprenant 200 µg/ml de lipase, on laisse agir les inhibiteurs pendant 45 min à température ambiante, on ajoute ensuite 5 µl (450ua) d'extrait brut standard dilué qu'on laisse réagir pendant 30 min à 37°C, puis on dépose 70 µl du mélange dans le puits de l'agar well test.

Les tampons utilisés pour les dilutions sont les suivants.
- pH 2,0: 100 mM d'acide maléique ajusté avec NaOH,
- pH 7,0: 100 mM de tampon phosphate (K₂HPO₄/KH₂PO₄),
- pH 7,5: 100 mM de tampon phosphate (K₂HPO₄/KH₂PO₄),
- pH 7,75 100 mM Tris.Cl.

On compare le diamètre du halo d'inhibition avec le diamètre témoin du halo obtenu sans addition d'enzyme qui pour chaque tampon et à chaque température d'incubation est d'environ 14 mm.

Le tableau I ci-après présente les résultats obtenus avec les enzymes testées. Dans ce tableau, l'enzyme est désignée par son type, le nom du fournisseur et le numéro d'article du fournisseur. L'inactivation de la bactériocine est indiquée en fonction de la concentration de l'enzyme ajoutée. Le chiffre 0 signifie qu'il n'y a plus de halo, autrement dit que l'activité antibactérienne de la présente bactériocine a été compromise par l'incubation avec l'enzyme. Le chiffre 14 indique qu'il y a encore un halo de 14mm correspondant à la pleine activité antibactérienne de la présente bactériocine.

**Tableau I**

| Enzymes | Concentration (µg/ml) | pH du tampon | Température d' incubation (°C) | Inactivation (mm) |
|---|---|---|---|---|
| Pepsine (SIGMA P-700) | 10 | 2,0 | 37 | 0 |
| Protéinase K (MERCK 1000 144) | 4 | 7,0 | 37 | 0 |
| Ficine (SIGMA F-3266) | 10 | 7,0 | 37 | 0 |
| Pronase E (SIGMA P-8038) | 10 | 7,5 | 37 | 0 |
| Nagarse (SIGMA P-4789) | 10 | 7,5 | 37 | 0 |
| Trypsine (SIGMA T-8128) | 10 | 7,5 | 25 | 0 |
| α-chymotrypsine (SIGMA C-7762) | 1 | 7,75 | 25 | 0 |
| Catalase (SIGMA C-10) | 10000 | 7,75 | 25 | 14 |
| α-amylase (SIGMA A-0521) | 1 | 7,75 | 25 | 14 |
| Lipase (SIGMA L-382) + inhibiteurs de protéases | 200 | 7,75 | 37 | 14 |

Toutes les protéases suppriment l'activité antibactérienne du surnageant, ce qui démontre qu'une partie protéique est impliquée dans cette activité.

Le fait que l'on n'observe aucune influence de la catalase sur l'activité antibactérienne des bactériocines, démontre aussi que l'inhibition de la croissance de la souche indicateur type n'est pas due à l'activité antibactérienne de H₂O₂ qui est connu pour avoir une activité semblable à celle des bactériocines, puisque H₂O₂ aurait été dégradé par la catalase.

De même, le fait que l'on n'observe pas d'inactivation de l'activité antibactérienne par l'α-amylase démontre l'absence de sucres hydrolysables par l'α-amylase impliqués dans cette activité antibactérienne.

En outre, le fait que la lipase ne présente pas d'influence sur l'activité antibactérienne, démontre aussi l'absence d'une fraction lipidique impliquée dans cette activité.

### Spectre d'inhibition

A l'aide de l'agar well test, sur agar inoculé avec diverses souches de spores ou bactéries, on détermine si le surnageant de culture de la souche CNCM I-1351 produisant les deux bactériocines selon l'invention, présente une activité inhibitrice de la croissance de ces diverses bactéries, en d'autres termes on détermine un spectre d'inhibition de ce surnageant.

Pour ce faire, on observe l'effet d'inhibition de la croissance de la souche testée produit par un échantillon de surnageant présentant une activité de 300 ua à pH 7,0, par rapport à l'effet, normalement nul, d'un même échantillon préalablement désactivé par une incubation à 37°C durant 30 min en présence de 5 µg/ml de protéinase K.

Pour réaliser ces essais, on utilise des plaques pour culture de tissu FALCON 3046 Multiwell. On recouvre 6 ml de milieu M17 contenant en outre 1% de lactose et 1,5% d'agar (milieu M17L) avec 700 µl de milieu M17 contenant en outre 1% de lactose et 0,6% d'agar inoculé avec 1% d'une culture de la souche à tester préparée durant la nuit précédente et diluée jusqu'à une DO₆₀₀ de 0,1.

Lorsque la souche à tester doit croître à partir de spores, on inocule avec 10⁵-10⁶ spores par ml de milieu de recouvrement.

Lorsque la souche à tester n'est pas un *Lactococcus*, un *Streptococcus* ou un *Enterococcus,* on remplace le milieu M17L par un milieu standard favorable à la croissance de la bactérie en question, notamment le milieu MRS comprenant en outre 2% de glucose pour les *Lactobacilli*, les *Pediococci*, les *Leuconostoc* et les *Bifidobacteria* (Sanofi Diagnostics Pasteur, France), le milieu RCM pour les spores ou les cellules végétatives de *Clostridia* (Oxoid, England), et le milieu BHI (Difco, USA) pour les autres bactéries testées.

On perce deux trous de 5 mm de diamètre et 5 mm de profondeur par plaque. On dépose dans un des trous un échantillon de 70 µl à 300 ua de la présente bactériocine, et dans l'autre un même échantillon préalablement désactivé. On incube à une température favorable à la croissance de la souche testée durant le temps nécessaire pour qu'elle recouvre la plaque d'un gazon bactérien visible.

On caractérise l'effet ou le degré d'inhibition par le diamètre du halo d'inhibition observé. On considère que l'inhibition est très forte (++++) si le halo présente un diamètre de 16-18 mm, forte (+++) pour un diamètre de 11,5-15,5 mm, moyenne (++) pour un diamètre de 7,5-11 mm, faible (+) pour un diamètre de 5-7,5 mm et nulle (-) si l'on n'observe aucun halo.

On teste ainsi plus de 74 souches de bactéries lactiques de différentes espèces et sous espèces et l'on constate qu'environ 7% seulement d'entre elles sont résistantes au surnageant. Le détail du résultat de ces tests est présenté dans le tableau II ci-après. Dans ce tableau II, comme dans les tableaux suivants, la désignation ou No de souche indiqué est le No qui lui est attribué dans la collection Nestlé (Adresse: NESTEC S.A., Centre de Recherche,Vers-chez-les-Blanc, CH-1000 Lausanne 26, Suisse). La température indiquée est la température d'incubation durant le test.

**Tableau II**

| Espèce | No | T (°C) | Inhibition |
|---|---|---|---|
| *Streptococcus thermophilus* | YS 3 | 42 | +++ |
| | YS4 | 42 | +++ |
| (Les souches Sfi 12 et 25 présentent une résistance à la souche CNCM I-1351 et un spectre d'action anti-bactéries similaire à cette souche) | YS11 | 42 | +++ |
| | YS7 | 42 | +++ |
| | YS8 | 42 | +++ |
| | YS20 | 42 | +++ |
| | Sfi3 | 42 | +++ |
| | Sfil8 | 42 | +++ |
| (La souche ST11 ne présente qu'une résistance à la souche CNCM I-1351; il semble cependant que moins de 5% des streptocoques soient capables d'exprimer une telle résistance) | Sfi19 | 42 | +++ |
| | Sfi20 | 42 | +++ |
| | Sfi16 | 42 | +++ |
| | ST11 | 42 | - |
| | Sfi12 | 42 | - |
| | Sfi25 | 42 | - |
| *Lactococcus lactis* (Productrices de nisine) | SL2 | 30 | ++ |
| | SL13 | 30 | ++ |
| | SL16 | 30 | ++ |
| | SL25 | 30 | ++ |
| | SL31 | 30 | ++ |
| | SL63 | 30 | ++ |
| *Lactococcus lactis* | SLP26 | 30 | ++ |
| | SLP29 | 30 | ++ |
| | SLP24 | 30 | ++ |
| | SL64 | 30 | ++ |
| | SL58 | 30 | ++ |
| | SL40 | 30 | ++ |
| *Lactococcus lactis biovar diacetylactis* | SD39 | 30 | ++ |
| | SD80 | 30 | ++ |
| | SD57 | 30 | ++ |
| | SD11 | 30 | ++ |
| | SD113 | 30 | ++ |
| *Lactococcus cremoris* | SC20 | 30 | ++ |
| | SC15 | 30 | ++ |
| | SC11 | 30 | ++ |
| | SC145 | 30 | ++ |
| | SC63 | 30 | ++ |
| | SC28 | 30 | ++ |
| *Enterococcus faecalis* | SFS 1 | 30 | + |
| | SFS 2 | 30 | + |
| | SFS 10 | 30 | + |
| *Enterococcus faecium* | SFM 1 | 30 | ++ |
| | SFM3 | 30 | ++ |
| | SFM 6 | 30 | ++ |
| | SFM 10 | 30 | ++ |
| | SFM14 | 30 | ++ |
| | SFM 9 | 30 | + |
| *Lactobacillus fermentum* | L26 | 30 | ++ |
| | L50 | 30 | ++ |
| | L28 | 30 | ++ |
| | LF16 | 30 | ++ |
| | LF15 | 30 | ++ |
| *Lactobacillus helveticus* | LH91 | 40 | ++++ |
| | LH2 | 40 | +++ |
| | LH3 | 40 | +++ |
| | LH1 | 40 | +++ |
| *Lactobacillus acidophilus* | LQ1 | 40 | ++ |
| | LQ3 | 40 | + |
| | LQ10 | 40 | ++ |
| | LQ21 | 40 | + |
| | LQ23 | 40 | ++++ |
| | LQ26 | 40 | |
| *Lactobacillus brevis* | LB2 | 30 | +++ |
| | LB10 | 30 | - |
| | LB13 | 30 | +++ |
| *Lactobacillus bulgaricus* | YL12 | 40 | ++++ |
| | YL2 | 40 | + |
| | YL5 | 40 | ++ |
| | LB32 | 40 | +++ |
| *Leuconostoc cremoris* | LCC1 | 30 | ++ |
| | LCC7 | 30 | ++ |
| | LCC2 | 30 | ++ |
| *Leuconostoc mesenteroides* | LCM9 | 30 | ++ |
| | LCM10 | 30 | ++ |
| | LCM18 | 30 | ++ |

Sur ce tableau II, on constate que le spectre d'inhibition du surnageant est étroit au sens que pour certaines espèces de *Lactobacilli*, telles que *Lactobacillus acidophilus, Lactobacillus brevis* et *Lactobacillus bulgaricus*, par exemple, le degré d'inhibition est hétérogène. Cependant, pour d'autres espèces telles que *L.fermentum, L.helveticus* et les *Lactococci* par exemple, le degré d'inhibition est homogène.

Ceci est intéressant au vu du fait qu'il est très difficile de distinguer une souche d'une autre dans une même espèce. On peut donc prévoir une utilisation intéressante du surnageant ou des bactériocines purifiées pour la distinction de souches industrielles.

On peut aussi prévoir l'utilisation d'une souche productrice d'au moins une des bactériocines selon la présente invention, en culture avec une autre souche de bactérie lactique naturellement résistante, ou un peu sensible, à la ou les bactériocines produites dans le milieu. On peut ainsi produire des yogourts, notamment des yogourts présentant une postacidification réduite, par exemple.

On constate également que le surnageant inhibe la croissance des six souches de *L. lactis* productrices de nisine. Ceci prouve que la présente bactériocine n'est pas la nisine. Ceci est confirmé par le fait que la présente bactériocine est inactivée par la trypsine à 10 µg/ml (cf Tableau I), ce qui n'est pas le cas de la nisine.

Cependant, le spectre d'inhibition du surnageant d'une culture produisant les deux bactériocines de l'invention, est également large au sens qu'il ne se limite pas aux espèces de bactéries lactiques mais qu'il s'étend à d'autres espèces de bactéries Gram positives, notamment aux bactéries alimentaires *Bifidobacteria*, aux bactéries indésirables ou pathogènes *Propionibacterium, Listeria innocua, Listeria monocytogenes* et *Micrococcus varians*, et aux spores et cellules de nombreuses bactéries pathogènes du genre *Clostridia* et *Bacilli*, par exemple, comme en font foi les résultats présentés dans le tableau III ci-après.

**Tableau III**

| Espèce | No | T (°C) | Inhibition |
|---|---|---|---|
| *Bifidobacterium breve* | BBR27 | 37 | +++ |
| | BBR4 | 37 | +++ |
| | BBR39 | 37 | +++ |
| *Bifidobacterium longum* | BL20 | 37 | +++ |
| | BL18 | 37 | +++ |
| | BL22 | 37 | +++ |
| *Bifidobacterium bifidum* | BB7 | 37 | +++ |
| | BB9 | 37 | +++ |
| | BB12 | 37 | +++ |
| *Bifidobacterium infantis* | BI6 | 37 | +++ |
| | BI1 | 37 | +++ |
| *Propionibacterium* | PP1 | 30 | +++ |
| *Clostridium botulinum* (Spores et cellules végétatives) | CB1 | 30 | ++ |
| | CB2 | 30 | ++ |
| *Clostridium tyrobutiricum* (Spores et cellules végétatives) | 107001 | 30 | + |
| | 107002 | 30 | ++ |
| Mélange de spores de | | | |
| *Clostridium sporogenes* | 100021 | | |
| *Clostridium fermentum* | 100022 | 30 | ++ |
| *Clostridium butilicum* (6 souches) | A-69; B-213; BKA40; B-73-211; A-80-124clovis; B-1-NCA | | |
| *Listeria innocua* | 24 | 30 | + |
| | 25 | 30 | + |
| | 27 | 30 | + |
| | 39 | 30 | + |
| | 40 | 30 | + |
| | 41 | 30 | + |
| *Listeria monocytogenes* | 57 | 30 | ++ |
| | 58 | 30 | ++ |
| | 59 | 30 | ++ |
| | 60 | 30 | ++ |
| | 61 | 30 | ++ |
| | 62 | 30 | ++ |
| *Bacillus subtilis* (Spores et cellules végétatives) | A2 | 30 | ++ |
| | A3 | 30 | ++ |
| | A13 | 30 | ++ |
| | A14 | 30 | ++ |
| | A15 | 30 | ++ |
| *Bacillus pumilus* (Spores et cellules végétatives) | B2 | 30 | ++ |
| *Bacillus cereus* (Spores et cellules végétatives) | C14 | 30 | ++ |
| *Micrococcus varians* | MCV1 | 30 | ++ |
| *Micrococcus luteus* (indicateur de nisine) | MCL1 | 30 | - |

Les résultats illustrés dans ce tableau III permettent entre autre de prévoir des utilisations intéressantes de ce surnageant ou des bactériocines purifiées, comme additif dans la préparation de produits alimentaires en tant qu'agent actif contre des agents pathogènes, notamment dans des produits de viande contre les *Clostridia*, dans des fromages contre *Listeria monocytogenes* et *C. tyrobutyricum*, ou dans des pâtes fraîches ou des sauces pour pâtes fraîches contre les *Bacilli* dont les souches ci-dessus tirent précisément leur origine, par exemple.

Enfin, les présentes bactériocines n'exercent aucun effet d'inhibition de la croissance de bactéries Gram-, comme on peut le constater au vu des résultats illustrés dans le tableau IV ci-après.

**Tableau IV**

| Espèce | No | T (°C) | Inhibition |
|---|---|---|---|
| *Escherichia coli* | BZ234 | 37 | - |
| *Salmonella thyphimurium* | 274 | 37 | - |
| | 273 | 37 | - |
| *Pseudomonas aeruginosa* | 5 | 37 | - |
| | 13 | 37 | - |
| *Pseudomonas fluorescens* | 11 | 37 | - |
| | 12 | 37 | - |

### Résistance à la chaleur, stabilité

Les bactériocines présentes dans l'extrait obtenu dans les conditions illustrées à l'exemple 1, ne présentent pas une bonne stabilité de conservation à 4°C, si l'extrait n'est pas préalablement chauffé. Par contre, elles présentent une bonne stabilité à la conservation, si l'extrait est chauffé brutalement durant au moins 15 min à 90-121°C, par exemple.

On a vérifié en particulier que plus de 50% de l'activité d'un tel extrait est conservée après 5 mois de conservation à 4°C si on a chauffé ledit extrait au préalable durant 20 min à 94°C dans un bain marie, par exemple. On a vérifié également que l'on conserve 100% de l'activité après avoir chauffé ledit extrait durant 60 min à 100°C (essai réalisé en bain d'huile thermostaté sur 1 ml du surnageant concentré ou non d'une culture d'une souche de *S*.*thermophilus* selon le présent procédé).

Par contre, les présentes bactériocines ne conservent qu'environ un tiers de leur activité après un traitement de stérilisation de 30 min à 121 °C (essai réalisé sur 40 ml du surnageant non concentré d'une culture d'une souche de *S.thermophilus* selon le présent procédé), par exemple.

Enfin, par des essais d'ultrafiltration sur filtres Amicon suivis d'une électrophorèse sur gel (SDS-PAGE), on constate que les bactériocines de l'invention présentes dans le surnageant d'une culture de *S. thermophilus*, notamment dans le surnageant de la culture standard obtenue à l'exemple 1, se présentent sous forme d'agrégats de poids moléculaire (PM) supérieur à 10 kDa, dont 67% présentent un PM inférieur à 100 kDa et 33% présentent un PM supérieur à 100 kDa.

### Purification des bactériocines

Dans la suite de la description, les pourcentages d'acide trifluoroacétique et d'acétonitrile sont donnés en volume.

On produit 1 litre d'une culture de la souche CNCM I-1351 dans un milieu M17 additionné de 1% de saccharose, pendant 6 h, à 42°C et en anaérobiose.

On ajoute alors directement à la culture 20 g de résine XAD-7 (Sigma), et l'on agite le tout doucement pendant 1 h à 4°C. On filtre ensuite le mélange à travers un filtre Schleicher & Schvell (Allemagne) n°604, puis on lave la résine retenue sur le filtre par 1 litre d'une solution d'acide acétique 50mM pH 5,2, afin d'éliminer les bactéries. On place alors la résine dans une colonne, et on élue les bactériocines par 45 ml d'une solution comprenant 70% d'acétonitrile et 0,1% d'acide trifluoroacétique (TFA). On obtient alors un éluat comprenant les deux bactériocines.

On sépare ensuite ces deux bactériocines éluées de la manière suivante.

On réduit premièrement le volume d'éluat jusqu'à 24 ml par une centrifugation /lyophilisation (Speedvac, Savant Instrument), on ajuste ensuite le volume obtenu à une concentration de NaCl 2M et Tris.Cl 250mM pH8, jusqu'à un volume de 50 ml, puis on injecte ce volume sur une colonne à intéraction hydrophobe Phényl Supérose HR 16/10 (Pharmacia) préalablement équilibrée par un tampon comprenant du Tris.Cl 50mM pH8 et du NaCl 2M. On fait ensuite passer à une vitesse de 4 ml/min, sucessivement, 200 ml du tampon précédent, 100 ml d'un gradient linéaire débutant avec le tampon précédent et terminant avec une solution Tris.Cl 50mM pH8, 100 ml de la solution précédente, 60 ml d'eau pure, 60 ml de la solution Tris.Cl 50mM pH8, et enfin 60 ml d'eau pure.

On dilue ensuite 50 µl de chaque fraction receuillie en sortie de colonne dans 50 µl de TFA 0,1%, puis on teste l'activité antibactérienne de chaque mélange par l'agar well test décrit ci-dessus.

On observe alors que les fractions du 470^{ième} au 490^{ième} ml présentent une activité antibactérienne. On mélange alors ces fractions, on réduit le volume de ce mélange par centrifugation/lyophilisation jusqu'à 1 ml, puis on injecte ce volume réduit sur une colonne Pep RPC HR 5/5 (Pharmacia) préalablement équilibrée par une solution de TFA 0,1%, appelée dans cette exposé "solution A". On prépare également une solution d'élution "B" comprenant 70% d'acétonotrile et 0,097% de TFA. On fait ensuite passer dans la colonne, à une vitesse de 1 ml/min, successivement, 1 ml de la solution A, 9 ml d'un gradient linéaire débutant par la solution A et terminant par un mélange 50/50 des solutions A et B, 2 ml de ce dernier mélange, 7 ml d'un gradient linéaire débutant par ce dernier mélange et terminant par un deuxième mélange 20/80 des solutions A et B, 2 ml d'un gradient linéaire débutant par ce deuxième mélange et terminant par la solution B, puis 2 ml de cette dernière solution.

On détermine alors l'activité antibactérienne des fractions en sortie de la colonne par l'agar well test, comme décrit précédemment. Toutes les fractions du 14^{ième} au 22^{ième} ml présentent une activité antibactérienne. Par contre, deux pics majoritaires de protéines, observés à une densité optique de 215nm, se distinguent dans les fractions 15 et 21 (en millilitre).

### Séquençage des bactériocines

On séquence la partie N-terminale des protéines comprises dans les fractions 15, 18, 20, 21 et 22 en utilisant un séquenceur automatique 4774 d'Applied Biosystems.

On révèle ainsi dans la fraction 15, la présence d'un peptide ayant une séquence de 48 acides aminés identique à celle, pour la partie N-terminale, de la séquence SEQ ID NO:1. Un autre peptide présent majoritairement dans la fraction 21 présente aussi une séquence de 23 acides aminés identique à celle, pour la partie N-terminale, de la séquence SEQ ID NO:2.

Ces résultats démontrent donc que la souche CNCM I-1351 produit deux peptides présentant une activité antibactérienne. Toutefois, l'aspect différent des halos d'inhibition obtenus entre les fractions 15 et 21, permet de suspecter une activité antibactérienne différente entre la thermophiline 1 et la thermophiline 2 de la présente invention.

D'un autre coté, on analyse la composition en acides aminés des fractions 15 et 21 hydrolysées au préalable par du HCl 6N, à 100°C pendant 24°C, par la méthode connue "de dérivativation au dabsylchloride". Les résultats montrent que la composition en acides aminés de chaque fraction semble déjà correspondre à leur séquence peptidique respective.

Enfin, on soumet également les fractions 15 et 21 à une spectromètrie de masse, et on révèle un poids moléculaire pour la thermophiline 1 qui est de l'ordre de 5800 Dalton, et un poids moléculaire pour la thermophiline 2 qui est de l'ordre de 3900 Dalton.

### Séquençage des gènes des bactériocines

On fabrique d'une manière classique les séquences nucléiques dégénérées SEQ ID NO:6 et SEQ ID NO:7 décrites dans la liste de séquence ci-après, qui correspondent respectivement à la partie N-terminale et la partie C-terminale du peptide de la thermophiline 1 séquencé précédemment.

On rend ensuite radioactif une partie du mélange de séquences SEQ ID NO:6, par l'action de la T4 polynucléotidekinase comme décrit dans le manuel de laboratoire "Molecular cloning, a laboratory manual" (second edition, Sambrook et al., Cold Spring Harbor, Laboratory Press, 1989), appelé dans le présent exposé "Maniatis".

On réalise ensuite une PCR ("polymerase chain reaction") à l'aide des deux mélanges non radioactifs de séquences dégénérées SEQ ID NO:6 et SEQ ID NO:7, sur une préparation d'ADN chromosomique de la souche CNCM I-1351, comme décrit dans le manuel "PCR technology" (H.A. Erdlich editor, M stockton press, London).

On révèle alors sur un gel d'électrophorèse une bande de 128 paires de bases (pb), qui est ensuite éluée selon Maniatis. Une partie est ensuite clonée directement dans le plasmide pGEM-T (Promega) en suivant les recommandations du fournisseur, puis est séquencée par la méthode des "didéoxynucléotides", selon Maniatis, en utilisant les sondes universelles de pUC19. On obtient ainsi une sonde ayant la séquence SEQ ID NO:8 décrite dans la liste de séquence ci-après, correspondant à une séquence codant pour les acides aminés 9 à 47 de la thermophiline 1. Enfin, l'autre partie de la bande éluée de 128 pb est rendue radioactive par la méthode appellée "random priming" selon Maniatis.

D'un autre coté, on réalise une digestion d'une préparation d'ADN chromosomique de la souche CNCM I-1351 par EcoRI et HindIII en suivant les recommandations du fournisseur d'enzyme, on fait ensuite migrer 10 µg de produit de digestion sur un gel d'électrophorèse analytique, on transfert en milieu alcalin l'ADN du gel sur une membrane "Zeta probe" (Biorad), on préhybride la membrane à 54°C pendant une nuit dans un milieu comprenant du SSC 6X, 1% de SDS et 1% de lait écrémé, puis on hybride cette membrane à la sonde dégénérée radioactive SEQ ID NO:6 dans le milieu d'hybridation précédent pendant premièrement 18 h à 54°C en diminuant la température de 2°C toutes les 3 h, puis pendant 24h à 42°C. On lave ensuite la membrane pendant 2 min, trois fois de suite, dans du SSC 6X à température ambiante, et pendant 1 min dans du SSC 6X à 47°C. On expose finalement la membrane sur un film d'autoradiographie. Toutes ces étapes sont effectuées selon le manuel Maniatis.

On révèle alors une bande de 3,6kb, qui nous permet de situer dans un gel préparatif d'électrophorèse de l'ADN chromosomique (300µg) de la souche CNCM I-1351 effectué dans les mêmes conditions que décrit ci-dessus, la partie de gel comprenant le morceau d'ADN qui nous intéresse. On découpe alors cette partie de gel que l'on élue d'une manière classique, et on ligue l'ADN élué au vecteur pUC19 (Messing et al., Methods Enzymol., 101:20, 1983) préalablement hydrolysé par EcoRI et HindIII. Ces étapes sont effectuées selon le manuel Maniatis.

On transforme ensuite classiquement par le milieu de ligation, la souche *Escherichia coli* BZ234 (collection du Biocentre, Université de Bâle, Suisse) rendue préalablement compétente. Les cellules transformées sont ensuite sélectionnées par α-complémentation. Puis, selon la méthode appelée "colony lift", selon Maniatis, on transfère 300 colonies transformées sur un filtre, on les lyse, on les hybride à la séquence radioactive SEQ ID NO:8, puis on expose le filtre sur un film d'autoradiographie.

On observe alors sur le film, 13 colonies ayant un plasmide capable de s'hybrider avec la séquence SEQ ID NO:8. On sélectionne alors deux des ces colonies, on en extrait classiquement l'ADN plasmidique, et l'on séquence par la méthode "des didéoxynucléotides" le fragment d'ADN cloné dans les deux plasmides pUC19 sélectionnés, à l'aide de sondes universelles de pUC19, puis de sondes basées sur les séquences ainsi obtenues.

On obtient ainsi une séquence nucléique SEQ ID NO:3 décrite dans la liste de séquences ci-après, qui est identique pour les deux plasmides sélectionnés. Cette séquence comprend ainsi un opéron codant pour deux protéines ayant les séquences en acides aminés SEQ ID NO:4 correspondant avant maturation à la thermophiline 1, et SEQ ID NO:5 correspondant avant maturation à la thermophiline 2 (voir la liste de séquences ci-après). Une troisième phase de lecture ouverte commence également à partir du nucléotide 679 de cette séquence, et doit certainement correspondre au gène d'immunité.

En comparant les séquences peptidiques N-terminales des bactériocines purifiées et les séquences en acides aminées des protéines codées par les phases codantes de l'opéron SEQ ID NO:3, on peut déterminer que la protéine de séquence en acide aminé SEQ ID NO:4 (thermophiline 1) présente un peptide leader de 23 acides aminés qui possède un motif Glycine-Glycine caractéristique d'une classe de bactériocines de bactéries lactiques. Enfin, la masse moléculaire de la thermophiline 1, calculée à partir de sa séquence nucléique, correspond à celle trouvée par spectrométrie, c'est à dire est de l'ordre de 5800 Dalton.

De même, la protéine de séquence en acide aminé SEQ ID NO:5 (thermophiline 2) présente un peptide leader de 21 acides aminés, qui possède un motif Glycine-Glycine caractéristique d'une classe de bactériocines de bactéries lactiques. Enfin, la masse moléculaire de la thermophiline 2, calculée à partir de sa séquence nucléique, correspond à celle trouvée par spectrométrie, c'est à dire est de l'ordre de 3900 Dalton.

### Rôle des différentes bactériocines

Une homologie avec le premier peptide de l'opéron de la "lactococein M" (Klaenhammer et al., FEMS Micro. Rew., 12, 39-86, 1993) a été trouvée pour la séquence de la thermophiline 1. Cette homologie conçerne la répétition d'un motif GA. De même, une homologie avec un gène de l'opéron "lactacin F" (Klaenhammer et al., cité ci-dessus) a été trouvée pour la thermophiline 2, dans la banque de donnée GenEMBL en utilisant le programme TFASTA de GCG.

Les deux opérons lactococein M et lactacin F codent en fait pour des complexes de poration faisant intervenir plusieurs peptides. Il est donc possible que l'opéron décrit précédemment puisse coder pour des peptides agissant conjointement en un complexe de poration.

Néanmoins, il n'est pas exclu que les deux bactériocines agissent indépendemment, du fait du halo d'inhibition quelque peu différent observé entre les deux thermophilines dans l'agar well test décrit précédemment.

Les exemples ci-après sont présentés à titre d'illustration du procédé de production et des utilisations de la bactériocine selon la présente invention. Les pourcentages y sont donnés en poids sauf indication contraire.

### Exemple 1

On inocule un milieu de culture M17 auquel on a ajouté 1% de saccharose avec 1% (^{V}/_{V}) d'une culture contenant 10⁸ germes de la souche de *S.thermophilus* CNCM I-1351 par ml. On incube durant 6 h à 42°C en anaérobiose après quoi le milieu contient environ 10⁸ germes de la souche par ml et il présente une DO₆₀₀ de 3,6.

On centrifuge la culture standard ainsi obtenue. On recueille le surnageant (standard). On l'acidifie à pH 1,5 avec H₃PO₄, on obtient un précipité que l'on écarte par centrifugation et l'on recueille un surnageant de précipitation acide.

On précipite les bactériocines contenues dans ce dernier avec 10% d'acide trichloracétique. On recueille les bactériocines précipitées et on les remet en suspension aqueuse avec de l'acide trifluoroacétique à 0,2% (^{V}/_{V}).

On reprécipite les bactériocines avec de l'acide trichloracétique à 10%. On recueille les bactériocine précipitées, on la lave avec de l'acétone à 100% et on les remet en suspension aqueuse avec de l'acide trifluoroacétique à 0,2% (^{V}/_{V}).

On obtient un extrait brut standard des présentes bactériocines présentant une activité de 1,4x10⁵ ua/ml.

Le tableau VI ci-après donne quelques précisions sur les caractéristiques d'un litre de surnageant standard et sur celles des 18 ml d'extrait brut standard, autrement dit de concentré que l'on en a tiré, notamment en termes de teneur en protéine et d'activité antibactérienne.

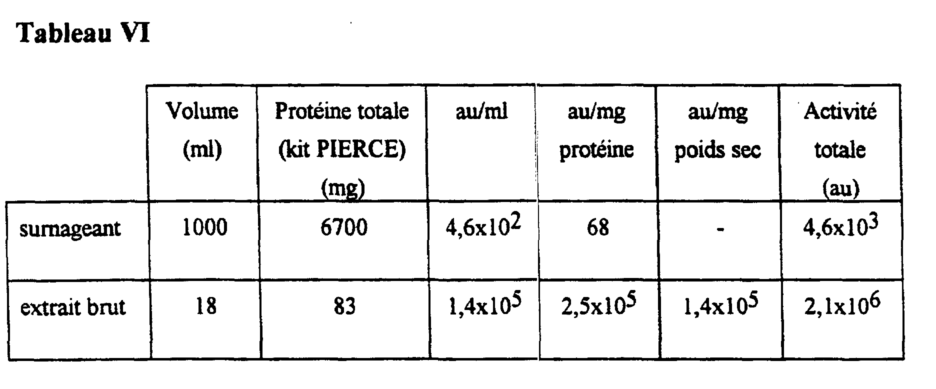

### Exemple 2

On prépare des yogourts étuvés comprenant la souche de l'invention *S. thermophilus* CNCM I-1351, et les souches *S. thermophilus* ST11 (résistante aux bactériocines selon l'invention mais ne présentant pas d'activité antibactérienne) et *L.bulgaricus* YL5 mentionnées précédemment.

On prépare ainsi un lait à base de lait entier comprenant 3,7% de matière grasse et 2,5% de poudre de lait écrémé. On pasteurise 40 l de ce lait à 92°C pendant 6 min, on l'homogénéise ensuite à 75°C et 150 bar (deux étages), enfin on le refroidit jusqu'à une température d'environ 42°C.

On réactive ensuite par plusieurs précultures successives dans un milieu MSK stérile (lait écrémé en poudre reconstitué à 10% comprenant 0,1% d'un extrait de levure commercial) les souches lyophilisées *S. thermophilus* CNCM I-1351, *S. thermophilus* ST11 1 et *L.bulgaricus* YL5.

On inocule ensuite le lait stérile à raison de 1% (^{V}/_{V}) de la troisième préculture de chaque souche *S. thermophilus* prise au stade de coagulation du milieu, et à raison de 2% (^{V}/_{V}) de la troisième préculture de la souche *L. bulgaricus* prise au stade de coagulation du milieu. On incube ensuite le lait à 42°C jusqu'à un pH d'environ 4,65, puis on le refroidit jusqu'à 4°C.

Pour comparaison, on réalise un yogourt traditionnel de la même manière que décrit ci-dessus, avec les souches décrites précédemment *S. thermophilus* YS8 et SFi3, et la souche *L.bulgaricus* YL18, qui sont traditionnellement utilisées pour la fabrication de yogourt.

Le tableau ci-après illustre les caractéristiques des produits obtenus, notamment leur pH pendant leur conservation à 4°C.

| Exemples | Temps d'acidification jusqu'à pH 4,65 | pH du produit au bout d'1 jour (à 4°C) | pH du produit au bout de 24 jours (à 4°C) |
|---|---|---|---|
| Exemple 2 | 8 h 30 | 4,6 | 4,6 |
| Exemple comparatif | 6 h | 4,34 | 4,3 |

### Exemple 3

On prépare du fromage mozzarella de manière traditionnelle à l'aide d'une culture *S.thermophilus* CNCM-1351.

### Exemple 4

On produit 10 litres d'une culture de la souche S. *thermophilus* CNCM I-1351 dans un milieu M17 additionné de 1% de saccharose, pendant 6 h, à 42°C et en anaérobiose. On ajoute alors directement à la culture 200 g de la résine XAD-7 (Sigma), et l'on agite le tout doucement pendant 1 h à 4°C. On filtre ensuite le mélange à travers un filtre Schleicher & Schvell (Allemagne) n°604, puis on lave la résine retenue sur le filtre par 10 litres d'une solution d'acide acétique 50mM pH 5,2, afin d'éliminer les bactéries. On ajoute alors à la résine 450 ml d'une solution comprenant de l'éthanol 100% et de l'acétate d'ammonium 20mM, on filtre le tout pour éliminer la résine, puis on lyophilise le filtrat jusqu'à obtenir une poudre comprenant les bactériocines selon l'invention, qui peut être utilisé dans l'industrie alimentaire.

On détermine alors l'activité antibactérienne de cette poudre préalablement diluée dans de l'eau, par l'agar well test décrit précédemment. Cette poudre présente 10⁷ ua/g de poudre.

Finalement, on ajoute à une mousse de viande, au cours de sa préparation de manière traditionnelle, 0,5 g/kg de la poudre ci-dessus. On obtient donc une mousse de viande comprenant 5x10³ ua/g de bactériocines capables d'inhiber totalement le développement de bactéries pathogènes, notamment les *Clostridia*.

### Exemple 5

Cet exemple conçerne la préparation d'une crème hydratante pour soins de la peau contenant 0,05 g/kg de la poudre décrite dans l'exemple 4, soit donc 5x10² ua/g de bactériocines capables d'inhiber le développement de bactéries indésirables sur la peau.

Pour fabriquer cette émulsion, on mélange les composants de la phase lipidique A et on la chauffe à 75°C. On prépare la phase aqueuse B, et on la chauffe également à 75°C, puis on l'ajoute à la phase lipidique A en brassant lentement, et on refroidit ensuite sous brassage lent jusqu'à température ambiante, soit environ 25°C. A cette température, on ajoute lentement les constituants C dans l'ordre de la formule.

| Phase lipidique A | % |
|---|---|
| Peg-6-stéarate, glycérate et peg-20-céthyl éther (peg:polyéthylène glycol) | 15 |
| Huile de vaseline | 5 |
| Huile de germe de blé stabilisée avec 0,1% de phénylindanes (antioxydant) et 1% de phospholipides de soja (voir EP94109355.1) | 3 |
| Huiles d'amandes douces | 2 |
| Alcool cétylique | 1 |
| Isostéaryl-isostéarate | 2 |
| 2-Octyl-docécyl-mystirate | 1 |
| Cire de lanoline | 1 |

| Phase aqueuse B | % |
|---|---|
| Méthylisothiazoline | 0,1 |
| Eau déminéralisée | 59,6 |
| Protéine de placenta humain | 2 |

| Additifs C | % |
|---|---|
| Propylèneglycol et extrait de calendula | 2 |
| 50% de collagène soluble dans de l'eau déminéralisée | 5,8 |
| Parfum | 0,3 |
| 2,5% de poudre de bactériocines selon l'ex.4 dans de l'eau déminéralisée | 0,2 |

### Exemple 6

On ajoute à une eau dentrifrice 0,5 g/kg de la poudre de bactériocines décrite à l'exemple 4. Ce dentifrice est ainsi capable d'inhiber le développement de bactéries pathogènes de la cavité buccale, et notamment *Streptococcus sobrinus*.

### Exemple 7

On pulvérise une solution comprenant la poudre de bactériocine de l'exemple 4 diluée dans de l'eau à raison de 1%, sur un produit alimentaire destiné à être stérilisé pour prévenir une post-contamination lors de l'emballage.

## Revendications

1. Bactériocine de *Streptococcus thermophilus* présentant la séquence en acides aminés SEQ ID NO:1

2. Bactériocine de *Streptococcus thermophilus* présentant la séquence en acides aminés SEQ ID NO:2.

3. Séquence nucléotidique codant pour l'une des bactériocines de *Streptococcus thermophilus* selon l'une des revendications 1 et 2.

4. Séquence selon la revendication 3, présentant la séquence nucléotidique SEQ ID NO:3 codant pour les bactériocines selon les revendications 1 et 2.

5. Séquence nucléotidique selon la revendication 4, **caractérisée en ce qu'**elle comprend les nucléotides 221 à 475 de la séquence SEQ ID NO:3.

6. Séquence nucléotidique selon la revendication 5, **caractérisée en ce qu'**elle comprend les nucléotides 289 à 475 de la séquence SEQ ID NO:3.

7. Séquence nucléotidique selon la revendication 4, **caractérisée en ce qu'**elle comprend les nucléotides 495 à 686 de la séquence SEQ ID NO:3.

8. Séquence nucléotidique selon la revendication 7, **caractérisée en ce qu'**elle comprend les nucléotides 558 à 686 de la séquence SEQ ID NO:3.

9. Séquence nucléotidique selon la revendication 5, **caractérisée en ce qu'**elle comprend les nucléotides 221 à 288 de la séquence SEQ ID NO:3.

10. Séquence nucléotidique selon la revendication 7, **caractérisée en ce qu'**elle comprend les nucléotides 495 à 557 de la séquence SEQ ID NO:3.

11. Peptide signal de *Streptococcus thermophilus* codé par les séquences nucléiques selon les revendications 9 et 10.

12. Souche de *Streptococcus thermophilus* CNCM I-1351 produisant au moins une des bactériocines selon les revendications 1 et 2.

13. Procédé de production d'au moins une bactériocine selon les revendications 1 et 2, dans lequel on cultive une souche de *Streptococcus thermophilus* productrice d'au moins une desdites bactériocines dans un milieu et dans des conditions favorables à la croissance de *Streptococcus thermophilus* jusqu'à ce que le milieu contienne de 10⁷-10⁹ germes de ladite souche par ml, on centrifuge la culture obtenue, puis on prépare un extrait du surnageant comprenant au moins une desdites bactériocines.

14. Procédé selon la revendication 13, dans lequel, pour préparer un extrait d'au moins une desdites bactériocines contenues dans ledit surnageant, on ajuste le pH du surnageant à 1,0-2,0 avec H₃PO₄, on écarte un précipité, et l'on réalise une ou plusieurs précipitations successives à l'acide trichloracétique suivies chacune d'une remise en suspension aqueuse à l'acide trifluoroacétique.

15. Procédé selon la revendication 13, dans lequel ladite souche de *Streptococcus thermophilus* est la souche CNCM I-1351 productrice desdites bactériocines.

16. Utilisation d'au moins une bactériocine selon les revendications 1 et 2, et/ou d'une souche de *Streptococcus thermophilus* productrice d'au moins une desdites bactériocines dans la préparation de produits alimentaires ou de produits cosmétiques.

17. Utilisation selon la revendication 16 d'une culture de ladite souche de *Streptococcus thermophilus* comme levain dans la préparation de fromages ou dans la préparation de laits acidifiés.

18. Utilisation selon la revendication 16 d'au moins une desdites bactériocines ou de ladite souche, comme additif ou agent actif contre des bactéries pathogènes, notamment dans la préparation de produits de viande tels que des mousses, comme agent actif contre *Clostridium botulinum*, ou dans la préparation de crèmes ou lotions, comme agent actif contre des bactéries pathogènes de la peau, ou dans la préparation de produits de l'hygiène buccale, comme agent actif contre des bactéries pathogènes de la cavité buccale, notamment contre *Streptococcus sobrinus*.

19. Utilisation selon la revendication 16 d'une bacteriocine sous forme d'extrait obtenu selon le procédé de la revendication 13.

20. Utilisation d'au moins une des séquences nucléique selon les revendications 3 à 8, pour conférer par transformation à des bactéries, des levures ou des plantes, la capacité d'inhiber certaines bactéries.

21. Utilisation d'une des séquences nucléiques selon les revendications 9 et 10, comme séqueace signal que l'on fusionne à un gène d'intérêt, pour conférer par transformation à une souche de *Streptococcus thermophilus* la capacité d'excréter la protéine codée par ledit gène d'intérêt.

## Claims

1. Bacteriocin of *Streptococcus thermophilus* having the amino acid sequence SEQ ID NO:1.

2. Bacteriocin of *Streptococcus thermophilus* having the amino acid sequence SEQ ID NO:2.

3. Nucleotide sequence coding for one of the bacteriocins of *Streptococcus thermophilus* according to either of claims 1 or 2.

4. Sequence according to claim 3, having the nucleotide sequence SEQ ID NO:3 coding for the bacteriocins according to claims 1 and 2.

5. Nucleotide sequence according to claim 4, **characterized in that** it comprises the nucleotides 221 to 475 of the sequence SEQ ID NO:3.

6. Nucleotide sequence according to claim 5, **characterized in that** it comprises the nucleotides 289 to 475 of the sequence SEQ ID NO:3.

7. Nucleotide sequence according to claim 4, **characterized in that** it comprises the nucleotides 495 to 686 of the sequence SEQ ID NO:3.

8. Nucleotide sequence according to claim 7, **characterized in that** it comprises the nucleotides 558 to 686 of the sequence SEQ ID NO:3.

9. Nucleotide sequence according to claim 5, **characterized in that** it comprises the nucleotides 221 to 288 of the sequence SEQ ID NO:3.

10. Nucleotide sequence according to claim 7, **characterized in that** it comprises the nucleotides 495 to 557 of the sequence SEQ ID NO:3.

11. Signal peptide of *Streptococcus thermophilus* coded by the nucleic sequences according to claims 9 and 10.

12. Strain of *Streptococcus thermophilus* CNCM I-1351 producing at least one of the bacteriocins according to claims 1 and 2.

13. Method for producing at least one bacteriocin according claims 1 and 2, wherein a strain of *Streptococcus thermophilus* producing at least one of the said bacteriocins is cultivated in a medium and under conditions favourable to the growth of *Streptococcus thermophilus* until the medium contains from 10⁷-10⁹ germs of the said strain per ml, the culture obtained is centrifuged and an extract of the supernatant is then prepared comprising at least one of the said bacteriocins.

14. Process according to claim 13, wherein in order to prepare an extract of at least one of the said bacteriocins contained in the said supernatant, the pH of the supernatant is adjusted to 1.0-2.0 with H₃PO₄, the precipitate is separated and one or more successive precipitations are carried out with trifluoroacetic acid, each one being followed by aqueous resuspension with trifluoroacetic acid.

15. Process according to claim 13, wherein the said strain of *Streptococcus thermophilus* is the strain CNCM I-1351 producing the said bacteriocins.

16. Use of at least one bacteriocin according to claims 1 and 2, and/or a strain of *Streptococcus thermophilus* producing at least one of the said bacteriocins in the preparation of food products or cosmetic products.

17. Use according to claim 16 of a culture of the said strain of *Streptococcus thermophilus* as a leaven in the preparation of cheeses or in the preparation of acidified milks.

18. Use according to claim 16 of at least one of the said bacteriocins or of the said strain, as an additive or active agent against pathogenic bacteria, in particular in the preparation of meat products such as mousses, as an active agent against *Clostridium botulinum*, or in the preparation of creams or lotions, as an active agent against pathogenic bacteria of the skin, or in the preparation of products for buccal hygiene, as an active agent against pathogenic bacteria of the buccal cavity, in particular against *Streptococcus sobrinus.*

19. Use according to claim 16 of a bacteriocin in the form of an extract obtained according to the method of claim 13.

20. Use of at least one of the nucleic sequences according to claims 3 to 8 in order to confer, by transformation, on bacteria, yeasts or plants, the capacity of inhibiting certain bacteria.

21. Use of one of the nucleic sequences according to claims 9 and 10, as a signal sequence which is fused with the relevant gene, to confer, by transformation, on a strain of *Streptococcus thermophilus* the capacity of excreting the protein coded by the said relevant gene.

## Patentansprüche

1. Bakteriocin aus *Streptcoccus thermophilus,* das die Aminosäuresequenz SEQ ID NO:1 aufweist.

2. Bakteriocin aus *Streptococcus thermophilus*, das die Aminosäuresequenz SEQ ID NO:2 aufweist.

3. Nukleotidsequenz, die für eines der Bakteriocine aus *Streptococcus thermophilus* nach einem der Ansprüche 1 und 2 kodiert.

4. Sequenz nach Anspruch 3, die die Nukleotidsequenz SEQ ID NO:3 aufweist, die für die Bakteriocine nach den Ansprüchen 1 und 2 kodiert.

5. Nukleotidsequenz nach Anspruch 4, **dadurch gekennzeichnet, daß** sie die Nukleotide 221 bis 475 der Sequenz SEQ ID NO:3 aufweist.

6. Nukleotidsequenz nach Anspruch 5, **dadurch gekennzeichnet, daß** sie die Nukleotide 289 bis 475 der Sequenz SEQ ID NO:3 aufweist.

7. Nukleotidsequenz nach Anspruch 4, **dadurch gekennzeichnet, daß** sie die Nukleotide 495 bis 686 der Sequenz SEQ ID NO:3 aufweist.

8. Nukleotidsequenz nach Anspruch 7, **dadurch gekennzeichnet, daß** sie die Nukleotide 558 bis 686 der Sequenz SEQ ID NO:3 aufweist.

9. Nukleotidsequenz nach Anspruch 5, **dadurch gekennzeichnet, daß** sie die Nukleotide 221 bis 228 der Sequenz SEQ ID NO:3 aufweist.

10. Nukleotidaequenz nach Anspruch 7, **dadurch gekennzeichnet, daß** sie die Nukleotide 495 bis 557 der Sequenz SEQ ID NO:3 aufweist.

11. Peptidsignal aus *Streptococcus thermophilus*, das von den Nukleinsäuresequenzen nach den Ansprüchen 9 und 10 kodiert wird.

12. Stamm von *Streptococcus thermophilus* CNCM I-1351, der wenigstens eines der Bakteriocine nach den Anspruchen 1 und 2 produziert.

13. Verfahren zur Herstellung von wenigstens einem Bakteriocin nach den Ansprüchen 1 und 2, bei dem man einen Stamm von *Streptococcus thermophilus*, der wenigstens eines der genannten Bakteriocine produziert, in einem Medium und unter Bedingungen kultiviert, die für das Wachstum von Streptococ*cus thermophilus* günstig sind, bis das Medium von 10⁷ bis 10⁹ Keime des genannten Stamms pro ml enthält, die erhaltene Kultur zentrifugiert, und danach einen Extrakt des Überstands herstellt, der wenigstens eines der genannten Bakteriocine enthält.

14. Verfahren nach Anspruch 13, bei dem man zur Herstellung eines Extrakts von wenigstens einem der genannten Bakteriocine, die in dem Überstand enthalten sind, den pH des Überstandes mit H₃PO₄ auf 1,0 bis 2,0 einstellt, einen Niederschlag abscheidet und eine oder mehrere nachfolgende Fällungen mit Trichloressigsäure durchführt, wobei man nach jeder mit Hilfe von Trifluoressigsäure wieder in wässrige Suspension bringt.

15. Verfahren nach Anspruch 13, bei dem der Stamm von *Streptococcus thermophilus* der Stamm CNCM I-1351 ist, der die genannten Bakteriocine produziert.

16. Verwendung wenigstens eines Bakteriocins aus den Ansprüchen 1 und 2 und/oder eines Stammes von *Streptococcus thermophilus*, der wenigetens eines der genannten Bakteriocine produziert, bei der Herstellung von Nahrungsmitteln oder Kosmetika.

17. Verwendung nach Anspruch 16 einer Kultur des Stamms von *Streptococcus thermophilus* als Ferment bei der Herstellung von Käsen oder bei der Herstellung von Sauermilchprodukten.

18. Verwendung nach Anspruch 16 von wenigstens einem der genannten Bakteriocine oder des genannten Stammes als Zusatz oder Wirkstoff gegen pathogene Bakterien, insbesondere bei der Herstellung von Fleischprodukten wie Schäumen, als Wirkstoff gegen *Chlostridium botulinum*, oder bei der Herstellung von Cremes oder Lotionen, als Wirkstoff gegen pathogene Bakterien der Haut, oder bei der Herstellung von Produkten für die Mundhygiene, als Wirkstoff gegen pathogene Bakterien der Mundhöhle, insbesondere gegen *Streptococcus sobrinus*.

19. Verwendung nach Anspruch 16 eines Bakteriocins in Form eines Extraktes, wie er bei dem Verfahren nach Anspruch 13 erhalten wird.

20. Verwendung von wenigstens einer der Nukleinsäuresequenzen nach den Ansprüchen 3 bis 8, um durch Transformation Bakterien, Hefen oder Pflanzen die Fähigkeit zu verleihen, bestimmte Bakterien zu inhibieren.

21. Verwendung einer der Nukleinsäuresequenzen nach den Ansprüchen 9 und 10 als Sequenzsignal, das man mit einem Gen von Interesse fusioniert, um durch Transformation einem Stamm von Streptococcus thermophilus die Fähigkeit zu verleihen, das kodierte Protein mit dem genannten Gen von Interesse zu exkretieren.
